(19) Europäisches Patentamt
European Patent Office
Office européen des brevets

(11) EP 3 106 473 B1

(12) **EUROPEAN PATENT SPECIFICATION**

(45) Date of publication and mention of the grant of the patent:
**03.04.2019 Bulletin 2019/14**

(21) Application number: **15860308.4**

(22) Date of filing: **18.11.2015**

(51) Int Cl.:
***C08F 136/06*** *(2006.01)* ***C08F 4/54*** *(2006.01)*

(86) International application number:
**PCT/KR2015/012424**

(87) International publication number:
**WO 2016/080764 (26.05.2016 Gazette 2016/21)**

# (54) CATALYST COMPOSITION FOR POLYMERIZATION OF CONJUGATED DIENE

KATALYSATORZUSAMMENSETZUNG ZUM POLYMERISIEREN VON KONJUGIERTEN DIENEN

COMPOSITION CATALYTIQUE POUR LA POLYMÉRISATION D'UN DIÈNE CONJUGUÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2014 KR 20140162933**
**20.11.2014 KR 20140162934**
**17.11.2015 KR 20150161323**

(43) Date of publication of application:
**21.12.2016 Bulletin 2016/51**

(73) Proprietor: **LG Chem, Ltd.**
**Seoul 07336 (KR)**

(72) Inventors:
- **KIM, Won Hee**
  **Daejeon 34122 (KR)**
- **BAE, Hyo Jin**
  **Daejeon 34122 (KR)**
- **AHN, Jeong Heon**
  **Daejeon 34122 (KR)**
- **JEON, Hee Jung**
  **Daejeon 34122 (KR)**
- **OH, Kyoung Hwan**
  **Daejeon 34122 (KR)**
- **CHO, Woo Jin**
  **Daejeon 34122 (KR)**
- **KANG, Suk Youn**
  **Daejeon 34122 (KR)**

(74) Representative: **Hoffmann Eitle**
**Patent- und Rechtsanwälte PartmbB**
**Arabellastraße 30**
**81925 München (DE)**

(56) References cited:
**EP-A1- 1 972 644** **EP-A2- 1 939 221**
**JP-A- 2000 034 320** **JP-A- 2013 194 099**
**KR-A- 20010 100 205** **KR-A- 20080 063 191**
**KR-A- 20120 031 186** **US-A1- 2014 213 433**

- **DAVID J WILSON: "Polymerization of 1,3-Butadiene using Aluminoxane-Based Nd-Carboxylate Catalysts", POLYMER INTERNATIONAL, GRANGEMOUTH, vol. 39, no. 3, 1 March 1996 (1996-03-01), pages 235-242, XP002535695,**
- **BULGAKOV R G ET AL: "Dehydration of LnCl3.6H2O (Ln=Tb, Nd, Dy) in the reaction with i-Bu3Al, Et3Al, Et2AlCl, EtAlCl2 and formation of the complexes LnCl3.3(BuO)3PO", JOURNAL OF ORGANOMETALLIC CHEMISTRY, ELSEVIER-SEQUOIA S.A. LAUSANNE, CH, vol. 636, no. 1-2, 25 November 2001 (2001-11-25), pages 56-62, XP004311758, ISSN: 0022-328X, DOI: 10.1016/S0022-328X(01)01166-4**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 106 473 B1

## Description

## TECHNICAL FIELD

### Cross-reference to Related Applications

This application claims priority to and the benefits of Korean Patent Application Nos. 10-2014-162933 and 10-2014-162934, filed with the Korean Intellectual Property Office on November 20, 2014, and Korean Patent Application No. 10-2015-161323, filed with the Korean Intellectual Property Office on November 17, 2015.

### Technical Field

The present invention relates to a catalyst composition for conjugated diene polymerization.

## BACKGROUND ART

With increasing demands for rubber compositions in various manufacturing fields such as tires, shoe soles or golf balls, values of conjugated diene-based polymers, particularly butadiene-based polymers among these, which are synthetic rubber, have increased as a substitute for natural rubber that faces product shortfall.

Generally, linearity or branching of conjugated diene-based polymers greatly affects physical properties of polymers. Specifically, melting rates and viscosity properties of polymers increase as linearity decreases and branching increases, and as a result, polymer processability is enhanced. However, when branching of polymers is high, molecular weight distribution becomes wide, and mechanical properties of the polymers affecting abrasion resistance, crack resistance, or a rebound property of a rubber composition decline.

In addition, linearity or branching of conjugated diene-based polymers, particularly butadiene-based polymers, is greatly influenced by the content of cis-1,4 bonds included in the polymer. Linearity increases as cis-1,4 bond content in a conjugated diene-based polymer increases, and as a result, the polymer has excellent mechanical properties and may enhance abrasion resistance, crack resistance and a rebound property of a rubber composition.

Accordingly, various methods for preparing a conjugated diene-based polymer having suitable processability while increasing linearity by increasing cis-1,4 bond content in the conjugated diene-based polymer have been researched and developed.

Specifically, a method using a polymerization system including a lanthanide rare earth element-containing compound, particularly a neodymium-based compound, has been proposed. However, conjugated diene-based polymers prepared through the method using the polymerization system do not have high cis-1,4 bond content, and therefore, physical property improving effects of a rubber composition were not sufficient.

In addition, a method for preparing a conjugated diene-based polymer by preforming a catalyst composition including an organic aluminum compound, a halogen compound and butadiene together with a neodymium-based compound, and carrying out a polymerization reaction of a conjugated diene-based monomer using the same has been proposed.

However, the method normally uses diisobutylaluminum hydride (DIBAH) as an aluminum-based compound capable of performing a scavenger role as well as alkylation and molecular weight control, and DIBAH included in a catalyst composition causes various problems during processes when preparing a conjugated diene-based polymer. In detail, in the above-mentioned method, preforming is carried out adding a small amount of butadiene in order to reduce the production of various active catalyst species in the alkylation step using DIBAH, and herein, a problem of processability decline occurs by polymers produced through the preforming of butadiene blocking a catalyst input line of a polymerization reactor. In addition, there is a problem in that molecular weights are not readily modified in the method, and it takes long until changes in the molecular weight control are identified. Particularly, conjugated diene-based polymers having many short chain branches and low linearity, that is, having an -S/R (stress/relaxation) value of less than 1 at 100°C are prepared since chain transfer often occurs during the polymerization reaction in the above-mentioned method. However, conjugated diene-based polymers having an -S/R value of less than 1 as above have a problem in that resistance properties, particularly rolling resistance (RR), of a rubber composition increases due to a high degree of branching, and fuel efficiency properties decline as a result.

In view of the above, development of methods capable of preparing conjugated diene-based polymers having high linearity through uniformization of active catalyst species, and quick and simple molecular weight control has been required.

## DISCLOSURE OF THE INVENTION

## TECHNICAL PROBLEM

An object of the present invention is to provide a catalyst composition for conjugated diene polymerization that does not cause problems during a process when used for preparing a conjugated diene-based polymer, exhibits excellent catalytic activity even with a small main catalyst amount, is capable of preparing a conjugated diene-based polymer having a high cis-1,4-bond content ratio and high linearity, and narrow molecular weight distribution by producing uniform active catalyst species, and is capable of reducing reaction time for polymerization, and a method for preparing the same.

## TECHNICAL SOLUTION

In view of the above, one aspect of the present invention provides a catalyst composition including a lanthanide rare earth element-containing compound which comprises a neodymium compound as defined in chemical formula 1 below; modified methylaluminoxane which is a compound of chemical formula below; a halogen compound; an aliphatic hydrocarbon-based solvent and wherein the catalyst composition does not comprise diisobutylaluminium hydride.

Another embodiment of the present invention provides a method for preparing the catalyst composition including mixing a lanthanide rare earth element-containing compound, modified methylaluminoxane, a halogen compound and an aliphatic hydrocarbon-based solvent, and then heat treating the result at a temperature of 0°C to 60°C.

Still another embodiment of the present invention provides a method for preparing the catalyst composition including mixing a lanthanide rare earth element-containing compound, modified methylaluminoxane and an aliphatic hydrocarbon-based solvent, first heat treating the result at a temperature of 10°C to 60°C, introducing a halogen compound to the resultantly obtained mixture, and second heat treating the result in a temperature range of 0°C to 60°C.

## ADVANSAGEOUS EFFECTS

A catalyst composition according to the present invention can exhibit excellent catalytic activity even with a small main catalyst amount without causing problems during a process when used for preparing a conjugated diene-based polymer. In addition, the catalyst composition is capable of preparing a conjugated diene-based polymer having a high cis-1,4-bond content ratio and high linearity, and narrow molecular weight distribution by producing uniform active catalyst species, and is capable of reducing reaction time for polymerization.

## MODE FOR CARRYING OUT THE INVENTION

Hereinafter, the present invention will be described in more detail in order to illuminate the present invention.

A term "preforming" used in the present specification means pre-polymerization in a catalyst composition for conjugated diene-based polymer preparation. Specifically, when a catalyst composition including a lanthanide rare earth element-containing compound, an aluminum compound and a halogen compound includes diisobutylaluminum hydride (DIBAH) as the aluminum compound, the catalyst composition also includes a small amount of monomers such as butadiene in order to reduce the possibility of various active catalyst species production. Accordingly, pre-polymerization of monomers such as butadiene is carried out in the catalyst composition for conjugated diene-based polymer preparation prior to a polymerization reaction for preparing a conjugated diene-based polymer, and this is referred to as preforming.

In addition, a term "premixing" used in the present specification means a state in which each constituent is uniformly mixed in a catalyst composition without being polymerized.

Existing catalyst systems for preparing a conjugated diene-based polymer are prepared by preforming a catalyst composition including a lanthanide rare earth element-containing compound, an aluminum compound such as diisobutylaluminum hydride (hereinafter, referred to as DIBAH), a halogen compound and butadiene. However, as described above, molecular weight is not readily modified when preparing a conjugated diene-based polymer using such a catalyst system, and it takes long until changes in the molecular weight control are identified. In addition, a problem of polymers produced by butadiene preforming blocking a catalyst input line of a polymerization reactor occurs during a process.

In view of the above, the present invention uses modified methylaluminoxane (hereinafter, referred to as 'MMAO') instead of an aluminum-based compound such as DIBAH used to be used for producing uniform active catalyst species in conjugated diene-based catalyst composition preparation, and accordingly, there is no concern for problems during a process, and superior catalytic activity is obtained since aliphatic hydrocarbon-based solvents may be used instead of commonly used aromatic hydrocarbon-based solvents. In addition, by introducing an aluminum-based compound including DIBAH alone separately from the catalyst composition when preparing a conjugated diene-based polymer using the same, uniform active catalyst species are capable of being produced, molecular weights are readily controlled, and as a result, a conjugated diene-based polymer having high linearity may be prepared.

In other words, a catalyst composition according to one embodiment of the present invention includes a lanthanide rare earth element-containing compound which comprises a neodymium compound of chemical formula 1; modified methylaluminoxane (MMAO) which is a compound of chemical formula 2; a halogen compound; an aliphatic hydrocarbon-based solvent and wherein the catalyst composition does not comprise diisobutylaluminium hydride.

Specifically, in the catalyst composition, the lanthanide rare earth element-containing compound may be a compound including any one, two or more elements among rare earth elements of atomic numbers 57 to 71 in the periodic table such as neodymium, praseodymium, cerium, lanthanum or gadolinium, and necessarily compound including neodymium according to the chemical formula 1.

In addition, the lanthanide rare earth element-containing compound may be a salt soluble in a hydrocarbon solvent such as carboxylates, alkoxides, β-diketone complexes, phosphates or phosphites of lanthanide rare earth elements, and herein, the hydrocarbon solvent may be saturated aliphatic hydrocarbon having 4 to 10 carbon atoms such as butane, pentane, hexane and heptane; saturated alicyclic hydrocarbon having 5 to 20 carbon atoms such as cyclopentane and cyclohexane; monoolefins such as 1-butene and 2-butene; aromatic hydrocarbon such as benzene, toluene and xylene; or halogenated hydrocarbon such as methylene chloride, chloroform, trichloroethylene, perchloroethylene, 1,2-dichloroethane, chlorobenzene, bromobenzene or chlorotoluene.

More specifically, the lanthanide rare earth element-containing compound comprises a neodymium compound of the following Chemical Formula 1:

[Chemical Formula 1]

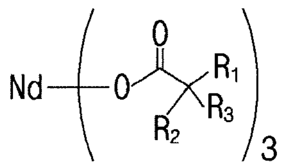

in Chemical Formula 1,

$R_1$ to $R_3$ are each independently a hydrogen atom, or a linear or branched alkyl group having 1 to 12 carbon atoms.

Specifically, the neodymium compound may be any one or a mixture of two or more selected from the group consisting of Nd(neodecanoate)$_3$, Nd(2-ethylhexanoate)$_3$, Nd(2,2-diethyl decanoate)$_3$, Nd(2,2-dipropyl decanoate)$_3$, Nd(2,2-dibutyl decanoate)$_3$, Nd(2,2-dihexyl decanoate)$_3$, Nd(2,2-dioctyl decanoate)$_3$, Nd(2-ethyl-2-propyl decanoate)$_3$, Nd(2-ethyl-2-butyl decanoate)$_3$, Nd(2-ethyl-2-hexyl decanoate)$_3$, Nd(2-propyl-2-butyl decanoate)$_3$, Nd(2-propyl-2-hexyl decanoate)$_3$, Nd(2-propyl-2-isopropyl decanoate)$_3$, Nd(2-butyl-2-hexyl decanoate)$_3$, Nd(2-hexyl-2-octyl decanoate)$_3$, Nd(2-t-butyl decanoate)$_3$, Nd(2,2-diethyl octanoate)$_3$, Nd(2,2-dipropyl octanoate)$_3$, Nd(2,2-dibutyl octanoate)$_3$, Nd(2,2-dihexyl octanoate)$_3$, Nd(2-ethyl-2-propyl octanoate)$_3$, Nd(2-ethyl-2-hexyl octanoate)$_3$, Nd(2,2-diethyl nonanoate)$_3$, Nd(2,2-dipropyl nonanoate)$_3$, Nd(2,2-dibutyl nonanoate)$_3$, Nd(2,2-dihexyl nonanoate)$_3$, Nd(2-ethyl-2-propyl nonanoate)$_3$ and Nd(2-ethyl-2-hexyl nonanoate)$_3$.

In addition, when considering excellent solubility for polymerization solvents without concern for oligomerization, a rate of conversion to an active catalyst species and superiority of catalytic activity improving effects obtained therefrom, the lanthanide rare earth element-containing compound may more specifically be a neodymium compound in which, in Chemical Formula 1, $R_1$ is a linear or branched alkyl group having 6 to 12 carbon atoms, and $R_2$ and $R_3$ are each independently a hydrogen atom or a linear or branched alkyl group having 2 to 8 carbon atoms, but $R_2$ and $R_3$ are not both hydrogen atoms at the same time. Specific examples thereof may include Nd(2,2-diethyl decanoate)$_3$, Nd(2,2-dipropyl decanoate)$_3$, Nd(2,2-dibutyl decanoate)$_3$, Nd(2,2-dihexyl decanoate)$_3$, Nd(2,2-dioctyl decanoate)$_3$, Nd(2-ethyl-2-propyl decanoate)$_3$, Nd(2-ethyl-2-butyl decanoate)$_3$, Nd(2-ethyl-2-hexyl decanoate)$_3$, Nd(2-propyl-2-butyl decanoate)$_3$, Nd(2-propyl-2-hexyl decanoate)$_3$, Nd(2-propyl-2-isopropyl decanoate)$_3$, Nd(2-butyl-2-hexyl decanoate)$_3$, Nd(2-hexyl-2-octyl decanoate)$_3$, Nd(2-t-butyl decanoate)$_3$, Nd(2,2-diethyl octanoate)$_3$, Nd(2,2-dipropyl octanoate)$_3$, Nd(2,2-dibutyl octanoate)$_3$, Nd(2,2-dihexyl octanoate)$_3$, Nd(2-ethyl-2-propyl octanoate)$_3$, Nd(2-ethyl-2-hexyl octanoate)$_3$, Nd(2,2-diethyl nonanoate)$_3$, Nd(2,2-dipropyl nonanoate)$_3$, Nd(2,2-dibutyl nonanoate)$_3$, Nd(2,2-dihexyl nonanoate)$_3$, Nd(2-ethyl-2-propyl nonanoate)$_3$ Nd(2-ethyl-2-hexyl nonanoate)$_3$, and among these, the neodymium compound may be any one or a mixture of two or more selected from the group consisting of Nd(2,2-diethyl decanoate)$_3$, Nd(2,2-dipropyl decanoate)$_3$, Nd(2,2-dibutyl decanoate)$_3$, Nd(2,2-dihexyl decanoate)$_3$ and Nd(2,2-dioctyl decanoate)$_3$.

Even more specifically, the lanthanide rare earth element-containing compound may be a neodymium compound in which, in Chemical Formula 1, $R_1$ is a linear or branched alkyl group having 6 to 8 carbon atoms, $R_2$ and $R_3$ are each independently a linear or branched alkyl group having 2 to 8 carbon atoms.

Thus, when the neodymium compound of Chemical Formula 1 includes a carboxylate ligand including an alkyl group with various lengths of 2 or more carbon atoms as a substituent at an α position, coagulation between the

compounds may be blocked by inducing stereoscopic changes around the neodymium central metal, and as a result, oligomerization may be suppressed. In addition, such a neodymium compound has high solubility for polymerization solvents, and has a high rate of conversion to an active catalyst species since the ratio of neodymium located in the central part having difficulties in being converted to an active catalyst species decreases.

Furthermore, the neodymium compound of Chemical Formula 1 may have solubility of approximately 4 g or greater per 6 g of a non-polar solvent at room temperature (20±5°C) . In the present invention, solubility of the neodymium compound means a level of being clearly dissolved without turbidity. By having such high solubility, excellent catalytic activity may be obtained.

Meanwhile, in the catalyst composition, the modified methylaluminoxane functions as an alkylating agent in the catalyst composition in place of existing DIBAH. The modified methylaluminoxane is a compound substituting a methyl group of methylaluminoxane with a modification group, specifically, a hydrocarbon group having 2 to 20 carbon atoms, and is a compound of the following Chemical Formula 2:

[Chemical Formula 2]

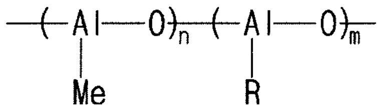

in Chemical Formula 2, R is a hydrocarbon group having 2 to 20 carbon atoms, m and n are each an integer of 2 or greater. In addition, Me in Chemical Formula 2 means a methyl group.

More specifically, R in Chemical Formula 2 may be a linear or branched alkyl group having 2 to 20 carbon atoms, a cycloalkyl group having 3 to 20 carbon atoms, an alkenyl group having 2 to 20 carbon atoms, a cycloalkenyl group having 3 to 20 carbon atoms, an aryl group having 6 to 20 carbon atoms, an aralkyl group having 7 to 20 carbon atoms, an alkylaryl group having 7 to 20 carbon atoms, an allyl group, or an alkynyl group having 2 to 20 carbon atoms, and more specifically, a linear or branched alkyl group having 2 to 10 carbon atoms such as an ethyl group, an isobutyl group, a hexyl group or an octyl group, and even more specifically an isobutyl group.

Even more specifically, the modified methylaluminoxane may be a compound substituting approximately 50 mol% or more of a methyl group of the methylaluminoxane, more specifically 50 mol% to 90 mol%, with a hydrocarbon group having 2 to 20 carbon atoms. When the content of the substituted hydrocarbon group in the modified methylaluminoxane is in the above-mentioned range, alkylation is facilitated and as a result, catalytic activity may increase.

Such modified methylaluminoxane may be prepared using common methods, and specifically, may be prepared using trimethylaluminum, and a trialkylaluminum other than trimethylaluminum. Herein, the trialkylaluminum may be triisobutylaluminum, triethylaluminum, trihexylaluminum, trioctylaluminum and any one or a mixture of two or more of these may be used. In this case, the modified methylaluminoxane may include trimethylaluminum; and a mixed alkyl group derived from one or more types of trialkylaluminums other than trimethylaluminum, and the trialkylaluminum may include any one or a mixture of two or more types selected from the group consisting of triisobutylaluminum, triethylaluminum, trihexylaluminum and trioctylaluminum.

With alkylaluminoxane such as methylaluminoxane (MAO) or ethylaluminoxane commonly used for conjugated diene polymer preparation, aromatic hydrocarbon-based solvents need to be used since alkylaluminoxane is not readily dissolved in aliphatic hydrocarbon-based solvents. However, aromatic hydrocarbon-based solvents have a problem of reducing reactivity, and when mixing an aromatic hydrocarbon-based solvent and an aliphatic hydrocarbon-based solvent in a catalyst system, there is a problem of reducing catalytic activity. However, in the present invention, modified methylaluminoxane capable of being readily dissolved in aliphatic hydrocarbon-based solvents is used, and accordingly, a single solvent system with an aliphatic hydrocarbon-based solvent such as hexane that is normally used as a polymerization solvent is capable of being used, which is more advantageous for a polymerization reaction. In addition, an aliphatic hydrocarbon-based solvent may facilitate catalytic activity, and reactivity may be further enhanced by such catalytic activity. As a result, molecular weights may be quickly and readily controlled, and polymerization is favorably progressed even at low temperatures due to very high catalytic activity, and time for polymerization reaction may be reduced even with a small main catalyst amount.

In addition, in the catalyst composition, specific examples of the aliphatic hydrocarbon-based solvent may include a mixed solvent of a linear, branched or cyclic aliphatic hydrocarbon-based solvent having 5 to 20 carbon atoms such as n-pentane, n-hexane, n-heptane, n-octane, n-nonane, n-decane, isopentane, isohexane, isopentane, isooctane, 2,2-dimethylbutane, cyclopentane, cyclohexane, methylcyclopentane or methylcyclohexane; or aliphatic hydrocarbon having 5 to 20 carbon atoms such as petroleum ether (or petroleum spirits) or kerosene, and any one or a mixture of two or more of these may be used. Among these, when considering excellent solubility for modified methylaluminoxane and superiority of catalytic activity improving effects resulted therefrom, the aliphatic hydrocarbon-based solvent may

be a linear, branched or cyclic aliphatic hydrocarbon-based solvent having 5 to 8 carbon atoms, or a mixture thereof, and more specifically n-hexane, cyclohexane, or a mixture thereof.

Furthermore, in the catalyst composition, the types of the halogen compound are not particularly limited, and those commonly used as halogenides in diene-based polymer preparation may be used without particular limit. Specifically, the halogen compound may include halogen simple substances, interhalogen compounds, halogenated hydrogen, organic halides, non-metal halides, metal halides, organic metal halides, and any one or a mixture of two or more of these may be used. Among these, when considering catalytic activity enhancement and superiority of reactivity improving effects resulted therefrom, any one or a mixture of two or more selected from the group consisting of organic halides, metal halides and organic metal halides may be used as the halogen compound.

More specifically, the halogen simple substance may include diatomic molecular compounds such as fluorine ($F_2$), chlorine ($Cl_2$), bromine ($Br_2$) or iodine ($I_2$).

Specific examples of the interhalogen compound may include iodine monochloride, iodine monobromide, iodine trichloride, iodine pentafluoride, iodine monofluoride, or iodine trifluoride.

In addition, specific examples of the halogenated hydrogen may include hydrogen fluoride, hydrogen chloride, hydrogen bromide, or hydrogen iodide.

Specific examples of the organic halide may include t-butyl chloride, t-butyl bromide, allyl chloride, allyl bromide, benzyl chloride, benzyl bromide, chloro-di-phenylmethane, bromo-di-phenylmethane, triphenylmethyl chloride, triphenylmethyl bromide, benzylidene chloride, benzyliene bromide, methyltrichlorosilane, phenyltrichlorosilane, dimethyldichlorosilane, diphenyldichlorosilane, trimethylchlorosilane, benzoyl chloride, benzoyl bromide, propionyl chloride, propionyl bromide, methyl chloroformate, methyl bromoformate, iodomethane, diiodomethane, triiodomethane (also called as 'iodoform'), tetraiodomethane, 1-iodopropane, 2-iodopropane, 1,3-diiodopropane, t-butyl iodide, 2,2-dimethyl-1-iodopropane (also called as 'neopentyl iodide'), allyl iodide, iodobenzene, benzyl iodide, diphenylmethyl iodide, triphenylmethyl iodide, benzylidene iodide (also called as 'benzal iodide'), trimethylsilyl iodide, triethylsilyl iodide, triphenylsilyl iodide, dimethyldiiodosilane, diethyldiiodosilane, diphenyldiiodosilane, methyltriiodosilane, ethyltriiodosilane, phenyltriiodosilane, benzoyl iodide, propionyl iodide, or methyl iodoformate.

Specific examples of the non-metal halide may include phosphorous trichloride, phosphorous tribromide, phosphorous pentachloride, phosphorous oxychloride, phosphorous oxybromide, boron trifluoride, boron trichloride, boron tribromide, silicon tetrafluoride, silicon tetrachloride, silicon tetrabromide, arsenic trichloride, arsenic tribromide, selenium tetrachloride, selenium tetrabromide, tellurium tetrachloride, tellurium tetrabromide, silicon tetraiodide, arsenic triiodide, tellurium tetraiodide, boron triiodide, phosphorous triiodide, phosphorous oxyiodide, or selenium tetraiodide.

Specific examples of the metal halide may include tin tetrachloride, tin tetrabromide, aluminum trichloride, aluminum tribromide, antimony trichloride, antimony pentachloride, antimony tribromide, aluminum trifluoride, gallium trichloride, gallium tribromide, gallium trifluoride, indium trichloride, indium tribromide, indium trifluoride, titanium tetrachloride, titanium tetrabromide, zinc dichloride, zinc dibromide, zinc difluoride, aluminum triiodide, gallium triiodide, indium triiodide, titanium tetraiodide, zinc diiodide, germanium tetraiodide, tin tetraiodide, tin diiodide, antimony triiodide or magnesium diiodide.

Specific examples of the organic metal halide may include dimethylaluminum chloride, diethylaluminum chloride, dimethylaluminum bromide, diethylaluminum bromide, dimethylaluminum fluoride, diethylaluminum fluoride, methylaluminum dichloride, ethylaluminum dichloride, methylaluminum dibromide, ethylaluminum dibromide, methylaluminum difluoride, ethylaluminum difluoride, methylaluminum sesquichloride, ethylaluminum sesquichloride, isobutylaluminum sesquichloride, methylmagnesium chloride, methylmagnesium bromide, ethylmagnesium chloride, ethylmagnesium bromide, n-butylmagnesium chloride, n-butylmagnesium bromide, phenylmagnesium chloride, phenylmagnesium bromide, benzylmagnesium chloride, trimethyltin chloride, trimethyltin bromide, triethyltin chloride, triethyltin bromide, di-t-butyltin dichloride, di-t-butyltin dibromide, di-n-butyltin dichloride, di-n-butyltin dibromide, tri-n-butyltin chloride, tri-n-butyltin bromide, methylmagnesium iodide, dimethylaluminum iodide, diethylaluminum iodide, di-n-butylaluminum iodide, diisobutylaluminum iodide, di-n-octylaluminum iodide, methylaluminum diiodide, ethylaluminum diiodide, n-butylaluminum diiodide, isobutylaluminum diiodide, methylaluminum sesquiiodide, ethylaluminum sesquiiodide, isobutylaluminum sesquiiodide, ethylmagnesium iodide, n-butylmagnesium iodide, isobutylmagnesium iodide, phenylmagnesium iodide, benzylmagnesium iodide, trimethyltin iodide, triethyltin iodide, tri-n-butyltin iodide, di-n-butyltin diiodide, or di -t-butyl tin diiodide.

The catalyst composition according to one embodiment of the present invention may include the above-mentioned constituents in optimum content so as to exhibit more superior catalytic activity in a polymerization reaction for forming a conjugated diene-based polymer.

Specifically, the catalyst composition may include the modified methylaluminoxane in a molar ratio of 5 to 200 and more specifically in a molar ratio of 10 to 100 with respect to 1 mol of the lanthanide rare earth element-containing compound.

In addition, the catalyst composition may include the halogen compound in a molar ratio of 1 to 10 and more specifically in a molar ratio of 2 to 6 with respect to 1 mol of the lanthanide rare earth element-containing compound.

Furthermore, the catalyst composition may include the aliphatic hydrocarbon-based solvent in a molar ratio of

20 to 20,000 and more specifically in a molar ratio of 100 to 1,000 with respect to 1 mol of the lanthanide rare earth element-containing compound.

More specifically, when considering superiority of catalytic activity for a polymerization reaction of a conjugated diene-based polymer, the catalyst composition according to one embodiment of the present invention is a pre-mixture including the modified methylaluminoxane in 5 mol to 200 mol, the halogen compound in 1 mol to 10 mol and the aliphatic hydrocarbon-based solvent in 20 mol to 20,000 mol with respect to 1 mol of the lanthanide rare earth element-containing compound, and herein, the lanthanide rare earth element-containing compound includes a neodymium compound in which, in Chemical Formula 1, $R_1$ is a linear or branched alkyl group having 6 to 12 carbon atoms, and $R_2$ and $R_3$ are each independently a hydrogen atom or a linear or branched alkyl group having 2 to 6 carbon atoms, but $R_2$ and $R_3$ are not both hydrogen atoms at the same time, and the modified methylaluminoxane is a compound substituting approximately 50 mol% or more of a methyl group of the methylaluminoxane with a hydrocarbon group having 2 to 20 carbon atoms, and the aliphatic hydrocarbon-based solvent includes any one or a mixture of two or more selected from the group consisting of linear, branched and cyclic aliphatic hydrocarbon-based solvents having 5 to 8 carbon atoms.

The catalyst composition having a composition as described above may exhibit catalytic activity of 10,000 kg[polymer]/mol[Nd]h during polymerization of 5 minutes to 60 minutes in a temperature range of 20°C to 90°C. The catalytic activity in the present invention is a value obtained from a molar ratio of the lanthanide rare earth element-containing compound, more specifically the neodymium compound of Chemical Formula 1, introduced with respect to the total yield of the prepared diene-based polymer.

Meanwhile, the catalyst composition according to one embodiment of the present invention is a pre-mixture of a lanthanide rare earth element-containing compound, MMAO, a halogen compound and an aliphatic hydrocarbon-based solvent, and may be prepared by mixing the lanthanide rare earth element-containing compound, the MMAO and the halogen compound in the aliphatic hydrocarbon-based solvent. Accordingly, another embodiment of the present invention provides a method for preparing the catalyst composition.

In preparing the catalyst composition, mixing of the lanthanide rare earth element-containing compound, the MMAO, the halogen compound and the aliphatic hydrocarbon-based solvent may be carried out using common methods.

In addition, in order to facilitate active catalyst species production in the mixing process, the mixing process may be carried out under a temperature condition of 0°C to 60°C. For this, a heat treatment process may be combined. More specifically, the lanthanide rare earth element-containing compound, the modified methylaluminoxane, and the aliphatic hydrocarbon-based solvent are mixed in the above-mentioned composition, the result is first heat treated at a temperature of 10°C to 60°C, and a second heat treatment may be carried out in a temperature range of 0°C to 60°C after introducing the halogen compound to the mixture resultantly obtained.

The catalyst composition prepared using the above-mentioned method exhibits excellent catalytic activity even with a small main catalyst amount, and may reduce reaction time of polymerization. In addition, a conjugated diene-based polymer having excellent catalytic activity and thereby having a high cis-1,4-bond content ratio, high linearity and narrow molecular weight distribution may be prepared, and unlike existing catalyst compositions for 1,4-cis-polybutadiene preparation, diisobutylaluminum hydride (DIBAH) is not included, and premixing instead of preforming is carried out, and therefore, it is very advantageous in terms of a process such that blockage of polymerization reactor catalyst input line by polymers caused by existing butadiene preforming may be prevented.

In addition, another embodiment of the present invention provides a method for preparing a conjugated diene-based polymer using the catalyst composition. Specifically, the method for preparing a conjugated diene-based polymer may include preparing a mixture of a chain transfer agent and a conjugated diene monomer as a monomer (step 1); and polymerization reacting the mixture using a catalyst composition including a lanthanide rare earth element-containing compound, modified methylaluminoxane, a halogen compound and an aliphatic hydrocarbon-based solvent (step 2).

When examining each step, the step 1 in the method for preparing a conjugated diene-based polymer according to one embodiment of the present invention is a step of preparing a mixture of a chain transfer agent and a conjugated diene-based monomer.

As described above, in the method for preparing a conjugated diene-based polymer according to one embodiment of the present invention, a chain transfer agent is separately mixed with a conjugated diene-based monomer instead of being introduced to a catalyst composition as in existing methods for preparing a conjugated diene-based polymer, and therefore, the molecular weight may be quickly controlled in a conjugated diene-based polymer production process, which leads to processability improvement.

In the step 1, organic aluminum compounds may be used as the Chain transfer agent.

Specific examples of the organic aluminum compound include trihydrocarbylaluminum such as trimethylaluminum, triethylaluminum, triisobutylaluminum, tri-n-propylaluminum, triisopropylaluminum, tri-n-butylaluminum, tri-t-butylaluminum, tri-n-pentylaluminum, trineopentylaluminum, tri-n-hexylaluminum, tri-n-octylaluminum, tris(2-ethylhexyl)aluminum, tricyclohexylaluminum, tris(1-methylcyclopentyl)aluminum, triphenylaluminum, tri-p-tolylaluminum, tris(2,6-dimethylphenyl)aluminum, tribenzylaluminum, diethylphenylaluminum, diethyl-p-tolylaluminum, diethylbenzylaluminum, ethyldiphenylaluminum, ethyldi-p-tolylaluminum or ethyldibenzylaluminum; or dihydrocarbylaluminum hydride such as

dimethylaluminum hydride, diethylaluminum hydride, di-n-propylaluminum hydride, diisopropylaluminum hydride, di-n-butylaluminum hydride, diisobutylaluminum hydride (DIBAH), di-t-butylaluminum hydride, dipentylaluminum hydride, dihexylaluminum hydride, dicyclohexylaluminum hydride or dioctylaluminum hydride, and any one or a mixture of two or more of these may be used.

In addition, as the Chain transfer agent, hydrogen; or silane compounds such as trimethyl silane, triethyl silane, tributyl silane, trihexyl silane, dimethyl silane, diethyl silane, dibutyl silane or dihexyl silane may be used. The silane compound may be used alone as the Chain transfer agent, or may be mixed with the organic aluminum compound described above. More specifically, when considering superiority of improving effects by the use of a Chain transfer agent, the Chain transfer agent may be diethylaluminum hydride, diisobutylaluminum hydride (DIBAH) or a mixture thereof among the above-mentioned compounds, and more specifically, may be diisobutylaluminum hydride.

The chain transfer agent not only controls molecular weights but may act as a scavenger, and therefore, the amount of the chain transfer agent used may vary depending on the amount of impurities and the amount of moisture. Specifically, in the preparation method according to one embodiment of the present invention, the content of the chain transfer agent capable of being used in the step 1 may be from 1 mol to 100 mol with respect to 1 mol of the lanthanide rare earth element-containing compound.

Meanwhile, in the step 1, the use of the monomer is not particularly limited as long as the monomer is commonly used in conjugated diene-based polymer preparation. Specifically, the monomer may include 1,3-butadiene, isoprene, 2,3-dimethyl-1,3-butadiene, 2-ethyl-1,3-butadiene, 2-methyl-1,3-pentadiene, 1,3-pentadiene, 3-methyl-1,3-pentadiene, 4-methyl-1,3-pentadiene, 1,3-hexadiene, 2,4-hexadiene, and more specifically, may be 1,3-butadiene or derivatives thereof such as 1,3-butadiene, isoprene, 2,3-dimethyl-1,3-butadiene or 2-ethyl-1,3-butadiene, and any one or a mixture of two or more of these may be used.

In addition, with the monomer, other monomers copolymerizable with the monomer may be selectively used. Herein, the other monomer additionally used may be used in proper content considering physical properties of a finally prepared conjugated diene-based polymer.

Specific examples of the other monomer may include aromatic vinyl monomers such as styrene, p-methylstyrene, $\alpha$-methylstyrene, 1-vinylnaphthalene, 3-vinyltoluene, ethylvinylbenzene, divinylbenzene, 4-cyclohexylstyrene and 2,4,6-trimethylstyrene, and any one or a mixture of two or more of these may be used. The other monomer may be used in the content of 20% by weight or less with respect to the total monomer weight used in a polymerization reaction for preparing a conjugated diene-based polymer.

In addition, in the method for preparing 1,4-cis polybutadiene according to one embodiment of the present invention, the step 2 is a step polymerization reacting the mixture prepared in the step 1 using a catalyst composition including a lanthanide rare earth element-containing compound; modified methylaluminoxane; a halogen compound and an aliphatic hydrocarbon-based solvent. Herein, the catalyst composition including a lanthanide rare earth element-containing compound, modified methylaluminoxane, a halogen compound and an aliphatic hydrocarbon-based solvent is the same as the catalyst composition described above.

The catalyst composition may include the lanthanide rare earth element-containing compound in an amount of 0.01 mmol to 0.25 mmol, specifically in 0.02 mmol to 0.20 mmol and more specifically in 0.02 mmol to 0.10 mmol with respect to 100 g of the conjugated diene-based monomer.

In addition, the catalyst composition includes the lanthanide rare earth element-containing compound in an amount of 0.01 mmol to 0.25 mmol, the modified methylaluminoxane in 0.1 mmol to 25.0 mmol, the halogen compound in 0.02 mmol to 1.5 mmol, and the aliphatic hydrocarbon-based solvent in 10 mmol to 180 mmol with respect to 100 g of the conjugated diene-based monomer.

More specifically, the catalyst composition includes the lanthanide rare earth element-containing compound in an amount of 0.01 mmol to 0.05 mmol, the modified methylaluminoxane in 0.1 mmol to 5.0mmol, the halogen compound in 0.03 mmol to 0.10 mmol, and the aliphatic hydrocarbon-based solvent in 10 mmol to 180 mmol with respect to 100 g of the conjugated diene-based monomer.

During the polymerization reaction in the step 2, a reaction terminating agent such as polyoxyethylene glycol phosphate, an antioxidant such as 2,6-di-t-butylparacresol, and additives such as a chelating agent, a dispersion agent, a pH controlling agent, a deoxidizer or an oxygen scavenger commonly used for facilitating solution polymerization may be further used selectively.

In addition, the polymerization reaction in the step 2 may be carried out in a temperature range of 20°C to 90°C, and particularly, a 100% conversion rate of polymers is capable of being accomplished in a short time even at a low temperature of 20°C to 30°C. When the temperature exceeds 90°C in the polymerization reaction, the polymerization reaction is difficult to be sufficiently controlled, and there is concern that cis-1,4 bond content of the produced diene-based polymer may decrease. When the temperature is less than 20°C, there is concern that polymerization reaction rate and efficiency may decrease.

Furthermore, according to the preparation method according to one embodiment of the present invention, the polymerization reaction may be carried out for 5 minutes to 60 minutes until the reaction reaches 1,4-cis polybutadiene

100% conversion, and specifically, may be carried out for 10 minutes to 30 minutes.

In addition, after the reaction is complete, the prepared conjugated diene-based polymer may be obtained by adding lower alcohols such as methyl alcohol or ethyl alcohol, or steam for precipitation. Accordingly, the method for preparing a conjugated diene-based polymer according to one embodiment of the present invention may further include precipitation and separation processes for a conjugated diene-based polymer prepared after the polymerization reaction. Herein, filtering, separating and drying processes for the conjugated diene-based polymer may be carried out using common methods.

According to the preparation method such as above, a conjugated diene-based polymer, specifically, a neodymium-catalyzed conjugated diene-based polymer including an active organic metal site derived from a catalyst including the lanthanide rare earth element-containing compound, more specifically the neodymium compound of Chemical Formula 1, and even more specifically, neodymium-catalyzed 1,4-cis polybutadiene including a 1,3-butadiene monomer unit is produced. In addition, the conjugated diene-based polymer may be 1,4-cis polybutadiene formed only with a 1,3-butadiene monomer.

In addition, 1,4-cis polybutadiene prepared using the above-mentioned preparation method has excellent physical properties including high linearity as described above. Consequently, another embodiment of the present invention provides a conjugated diene-based polymer prepared according to the preparation method described above.

Specifically, the conjugated diene-based polymer is a polymer having high linearity with a -S/R (stress/relaxation) value of 1 or greater at 100°C. More specifically, a -S/R value of the conjugated diene-based polymer is from 1 to 1.2, and even more specifically from 1.045 to 1.2.

In the present invention, the -S/R value represents changes in stress shown as a reaction for the same amount of strain generated in a material, and is an index representing polymer linearity. A lower -S/R value commonly means lower conjugated diene-based polymer linearity, and as linearity decreases, rolling resistance increases when used in a rubber composition. In addition, a degree of branching and molecular weight distribution may be predicted from the -S/R value. As the -S/R value decreases, the degree of branching increases, and the molecular weight distribution becomes wider, and as a result, mechanical properties are poor whereas polymer processability is superior.

By the conjugated diene-based polymer according to one embodiment of the present invention having the above-mentioned -S/R value range, rolling resistance (RR) decreases compared to polymers prepared using existing catalyst systems, and an effect of greatly enhancing fuel efficiency properties may be obtained.

In the present invention, the -S/R value may be measured using a Mooney viscometer, for example, a Large Rotor of MV2000E manufactured by Monsanto under a condition of 100°C and Rotor Speed 2±0.02 rpm. Specifically, the polymer is left unattended for 30 minutes or longer at room temperature (23±5°C), 27±3 g thereof is collected and inside a die cavity is filled with the polymer sample, and Mooney viscosity is measured while operating a Platen and applying Torque, and by measuring a slope of Mooney viscosity changes appearing while releasing Torque, the - S/R value may be determined.

In addition, the conjugated diene-based polymer according to one embodiment of the present invention may have narrow molecular weight distribution having polydispersity (PDI) of 3 or less. When the conjugated diene-based polymer has PDI of greater than 3, there is concern that mechanical properties such as abrasion resistance and impact resistance decline when used in a rubber composition. When considering the significance of mechanical property improving effects of the polymer due to PDI control, PDI of the conjugated diene-based polymer may be specifically from 2.0 to 2.5, and more specifically from 2.35 to 2.5.

In the present invention, PDI of a conjugated diene-based polymer is also referred to as molecular weight distribution (MWD), and may be calculated from a ratio (Mw/Mn) of a weight average molecular weight (Mw) to a number average molecular weight (Mn). Herein, the number average molecular weight (Mn) is a common average of individual molecular weights of polymers calculated by measuring molecular weights of n polymer molecules, and dividing the sum of these molecular weights by n, and the weight average molecular weight (Mw) represents molecular weight distribution of a polymer composition, and may be calculated by the following Mathematical Formula 1.

[Mathematical Formula 1]

$$M_w = \frac{\sum_i N_i M_i^2}{\sum_i N_i M_i}$$

In Mathematical Formula 1, Ni is the number of molecules having a molecular weight of Mi. An average of all molecular weights may be represented by gram per mol (g/mol).

Furthermore, in the present invention, the weight average molecular weight and the number average molecular

weight are each a polystyrene converted molecular weight analyzed with gel permeation chromatography (GPC).

In addition, the conjugated diene-based polymer according to one embodiment of the present invention may have a weight average molecular weight (Mw) of 400,000 g/mol to 2,500,000 g/mol and specifically 1,100,000 g/mol to 2,300,000 g/mol while satisfying the polydispersity condition. Furthermore, the conjugated diene-based polymer according to one embodiment of the present invention may have a number average molecular weight (Mn) of 100,000 g/mol to 1,000,000 g/mol and specifically 500,000 g/mol to 900,000 g/mol. When the weight average molecular weight (Mw) of the conjugated diene-based polymer is less than 400,000 g/mol or the number average molecular weight (Mn) is less than 100,000 g/mol, there is concern of an increase in hysteresis loss due to elasticity decline of a vulcanizate, and degeneration of abrasion resistance. In addition, when the weight average molecular weight (Mw) is greater than 2,500,000 g/mol or the number average molecular weight (Mn) is greater than 1,000,000 g/mol, processability of the conjugated diene-based polymer declines causing degeneration in the workability of a rubber composition, and mixing and kneading become difficult, and as a result, physical properties of the rubber composition may be difficult to be sufficiently enhanced.

Furthermore, the conjugated diene-based polymer according to one embodiment of the present invention may have Mooney viscosity (MV) of 30 to 90 and specifically 70 to 90 at 100°C. More superior processability may be obtained when the Mooney viscosity is in the above-mentioned range.

In the present invention, Mooney viscosity may be measured using a Mooney viscometer, for example, a Large Rotor of MV2000E of Monsanto at 100°C and Rotor Speed 2±0.02 rpm. Herein, the measurement may be made by leaving the sample used unattended for 30 minutes or longer at room temperature (23±5°C), collecting 27±3 g thereof, and filling inside a die cavity with the sample, and operating a Platen.

In addition, in the conjugated diene-based polymer according to one embodiment of the present invention, cis bond content in the conjugated diene-based polymer measured using Fourier Transform Infrared Spectroscopy, specifically cis-1,4 bond content, may be 95% or greater and more specifically 96% or greater. When the cis-1,4 bond content in the polymer is high as above, linearity increases, and abrasion resistance and crack resistance of a rubber composition may be enhanced when being mixed to the rubber composition.

Accordingly, another embodiment of the present invention provides a rubber composition including the conjugated diene-based polymer.

Specifically, the rubber composition may include the conjugated diene-based polymer in 10% by weight to 100% by weight and a rubber component in 0 to 90% by weight. When the content of the conjugated diene-based polymer is less than 10% by weight, effects of improving abrasion resistance, crack resistance and ozone resistance of the rubber composition may be insignificant.

In the rubber composition, the rubber component may be specifically natural rubber (NR); or synthetic rubber such as a styrene-butadiene copolymer (SBR), hydrogen-added SBR, polybutadiene (BR) having low cis-1,4-bond content, hydrogen-added BR, polyisoprene (IR), butyl rubber (IIR), ethylene-propylene rubber, ethylene-propylene diene rubber, polyisobutylene-co-isoprene, neoprene, poly(ethylene-co-propylene), poly(styrene-co-butadiene), poly(styrene-co-isoprene), poly(styrene-co-isoprene-co-butadiene), poly(isoprene-co-butadiene), poly(ethylene-co-propylene-co-diene), polysulfide rubber, acrylic rubber, urethane rubber, silicone rubber or epichlorohydrin rubber, and any one or a mixture of two or more of these may be used.

In addition, the rubber composition may further include a filler in 10 parts by weight or greater with respect to 100 parts by weight of the rubber component. Herein, the filler may be carbon black, starch, silica, aluminum hydroxide, magnesium hydroxide, clay (hydrated aluminum silicate), and any one or a mixture of two or more of these may be used.

Furthermore, the rubber composition may further include, in addition to the rubber component and the filler described above, compounding agents commonly used in a rubber industry such as a vulcanizing agent, a vulcanization accelerator, an antiaging agent, an antiscorching agent, a softner, zinc oxide, stearic acid or silane coupling agent by properly selecting and mixing them within a range that does not undermine an object of the present invention.

Specifically, such a rubber composition is useful for preparing various molded rubber articles such as automobiles, trucks (tracks), tires for buses (for example, tire treads, side walls, sub-treads, bead fillers, brake members), elastic components of a tire stock, O-rings, profiles, gaskets, films, hoses, belts, shoe soles, cushion rubber or window seals. Particularly, by including a conjugated diene-based polymer having high linearity with a -S/R value of 1 or greater at 100°C, resistance properties, particularly rolling resistance, decreases, and significantly improved fuel efficiency properties are obtained, and as a result, the rubber composition may be useful in tires requiring low resistance properties and excellent fuel efficiency properties.

Hereinafter, the present invention will be described in detail with reference to examples in order to specifically describe the present invention. The examples of the present invention are provided in order to more completely describe the present invention for those skilled in the art.

[Preparation of Neodymium Compound]

Preparation Example 1: Synthesis of Nd(2,2-dihexyl decanoate)

To a 50 ml round flask having 0.35 g (1.0 mmol) of 2,2-dihexyl decanoic acid therein, 10 ml of ethanol was added, and the result was stirred for 10 minutes at room temperature (20±5°C). 1.0 ml of a 1.0 M aqueous sodium hydroxide solution (1.0 mmol) was added to the mixed solution obtained as a result, and the result was stirred for 1 hour at room temperature (20±5°C) to prepare a first mixed solution.

A second mixed solution was prepared by placing 0.125 g (0.35 mmol) of neodymium chloride hydrate in a 250 ml round flask, and then adding 20 ml of hexane and 10 ml of ethanol thereto to dissolve the neodymium compound.

The first mixed solution was introduced to a dropping funnel and was dropped to the second mixed solution at room temperature (20±5°C) to prepare a third mixed solution. After completing the addition, the result was stirred for 15 hours at room temperature (20±5°C).

The third mixed solution was vacuum distilled to remove all the solvent, 50 ml of hexane and 50 ml of distilled water were added to the third mixed solution, the result was introduced to a separatory funnel, and the organic layer was extracted repeating 3 times. Sodium sulfate was added to the collected organic layer, the result was stirred for 10 minutes at room temperature (20±5°C), and then the solution obtained from filtration was removed by vacuum distillation. As a result, 0.38 g (yield 94%) of title compound (I), which is yellow and blue solid, dissolved in hexane was obtained.

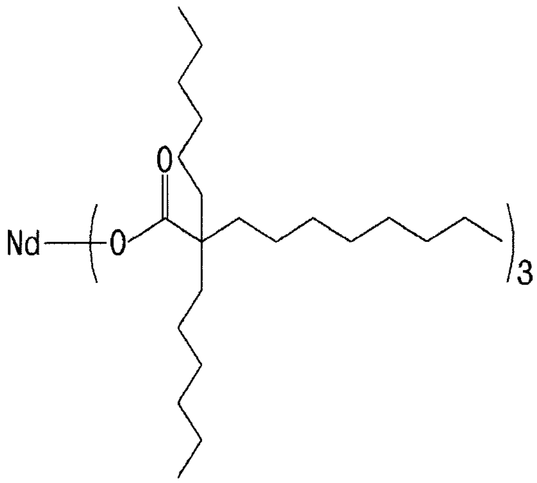

(I)

FT-IR: u 953, 2921, 2852, 1664, 1557, 1505, 1457, 1412, 1377, 1311, 1263 $cm^{-1}$

Preparation Example 2: Synthesis of $Nd(neodecanoate)_3$

To a 100 ml round flask having 4.32 g (25 mmol) of neodecanoic acid therein, 100 ml of ethanol was added, and the result was stirred for 10 minutes at room temperature (20±5°C). 25 ml of a 1.0 M aqueous sodium hydroxide solution (25 mmol) was added to this solution, and the result was stirred for 1 hour at room temperature (20±5°C) to prepare a first mixed solution.

A second mixed solution was prepared by placing 3.0 g (8.3 mmol) of neodymium chloride hydrate in a 500 ml round flask, and then adding 150 ml of hexane and 100 ml of ethanol thereto to dissolve the neodymium compound.

The first mixed solution was introduced to a dropping funnel and was dropped to the second mixed solution at room temperature (20±5°C) to prepare a third mixed solution. After completing the addition, the result was stirred for 15 hours at room temperature (20±5°C).

The third mixed solution was vacuum distilled to remove all the solvent, 100 ml of hexane and 100 ml of distilled water were added to the third mixed solution, the result was introduced to a separatory funnel, and the organic layer was extracted repeating 3 times. Sodium sulfate was added to the collected organic layer, the result was stirred for 10 minutes at room temperature (20±5°C), and then the solution obtained from filtration was removed by vacuum distillation. As a result, 5.3 g (yield: 96%) of a title compound (II), which is purple solid, was obtained.

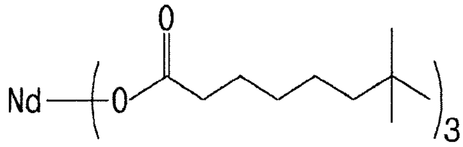

(II)

FT-IR: u 956, 2926, 2872, 1512, 1462, 1411, 1375, 1181, 641 $cm^{-1}$

[Preparation of Conjugated Diene-based Polymer]

Example 1

Step (i): Preparation of chain transfer agent and Conjugated Diene-Based Monomer Mixture

Vacuum and nitrogen were alternately applied to a completely dried 10 L high pressure reactor, and an atmospheric pressure (1±0.05 atm) state was made by filling the reactor with nitrogen again. To this high pressure reactor, hexane (2086.4 g) and 1,3-butadiene (250 g) were added and mixed, and first heat treatment was carried out for approximately 10 minutes at 70°C. Diisobutylaluminum hydride (DIBAH) was added and mixed to this high pressure reactor in an amount listed in the following Table 1, the resultant mixed solution was second heat treated for approximately 2 minutes at approximately 70°C to prepare a mixture of a chain transfer agent and a conjugated diene-based monomer.

Step (ii): Polymerization Reaction

The neodymium compound of Preparation Example 1, modified methylaluminoxane (MMAO)(MISC MAO, Lot: 9578-110-3, Albemarle Corporation, Al content in isoheptane=8.6% by weight) and hexane were premixed in amounts listed in the following Table 1, and then the result was heat treated for 10 minutes at 50°C. To the resultant mixture, diethylaluminum chloride (DEAC) was added in an amount listed in the following Table 1, and the result was heat treated for 10 minutes at 26°C to prepare a catalyst composition.

To the mixture of the chain transfer agent and the conjugated diene-based monomer prepared in the step (i), the catalyst composition was injected, and a polymerization reaction was carried out for 40 minutes at 70°C to obtain 1,4-cis polybutadiene.

Example 2

1,4-Cis polybutadiene was prepared in the same manner as in Example 1 except that the neodymium compound prepared in Preparation Example 1, the MMAO, the hexane, the DIBAH and the DEAC were used in amounts listed in the following Table 1.

Examples 3 and 4

1,4-Cis polybutadiene was prepared in the same manner as in Example 1 except that the neodymium compound prepared in Preparation Example 2 was used instead of the neodymium compound prepared in Preparation Example 1, and the neodymium compound of Preparation Example 2, the MMAO, the hexane, the DIBAH and the DEAC were used in amounts listed in the following Table 1.

Examples 5 to 7

1,4-Cis polybutadiene was prepared in the same manner as in Example 1 except that the neodymium compound prepared in Preparation Example 2 was used instead of the neodymium compound prepared in Preparation Example 1, and the polymerization reaction was carried out for approximately 40 minutes at a polymerization reaction temperature of 30°C using the neodymium compound of Preparation Example 2, the MMAO, the hexane, the DIBAH and the DEAC in amounts listed in the following Table 1.

Comparative Example 1

Vacuum and nitrogen were alternately applied to a completely dried 10 L high pressure reactor, and an atmospheric pressure state was made by filling the reactor with nitrogen again. To this high pressure reactor, hexane (2086.4

g) and 1,3-butadiene (250 g) were added and mixed, and first heat treatment was carried out for approximately 10 minutes at 70°C. A solution mixing the neodymium compound of Preparation Example 1, DIBAH and DEAC in amounts listed in the following Table 1 was added to this high pressure reactor, and the result was polymerization reacted for 30 minutes at 70°C to prepare 1,4-cis polybutadiene.

Comparative Example 2

1,4-Cis polybutadiene was prepared in the same manner as in Comparative Example 1 except that the neodymium compound prepared in Preparation Example 2 was used instead of the neodymium compound prepared in Preparation Example 1, and the neodymium compound of Preparation Example 2, the hexane, the DIBAH and the DEAC were used in amounts listed in the following Table 1, and the reaction was carried out under a condition listed in Table 1.

Comparative Examples 3 and 4

1,4-Cis polybutadiene was prepared in the same manner as in Comparative Example 1 except that the neodymium compound prepared in Preparation Example 2 was used instead of the neodymium compound prepared in Preparation Example 1, and the neodymium compound of Preparation Example 2, the hexane, the DIBAH and the DEAC were used in amounts listed in the following Table 2, and the reaction was carried out under a condition listed in Table 2.

Test Example 1: Evaluation on Conversion Rate and Catalytic Activity

After completing the polymerization reaction for preparing 1,4-cis polybutadiene in the examples and the comparative examples, some of the reaction solution was taken to measure a conversion rate, and catalytic activity was calculated based on the conversion rate.

In detail, the conversion rate was calculated using a ratio of a value measuring the mass of some of the reaction solution taken after completing the polymerization reaction, and a value measuring the mass of polybutadiene remaining after removing all the hexane solvent and residual butadiene by heating the some of the polymer for 10 minutes at 120°C.

In addition, catalytic activity was calculated based on the conversion rate using the mass of the produced polybutadiene, the number of mol of the neodymium compound used in the polymerization reaction, and the polymerization time. The results are shown in the following Tables 1 and 2.

Test Example 2: Evaluation on Physical Property

Physical properties of each 1,4-cis polybutadiene prepared in the examples and the comparative examples were measured as follows, and the results are shown in the following Tables 1 and 2.

1) Weight Average Molecular Weight (Mw), Number Average Molecular Weight (Mn) and Polydispersity (PDI)

The 1,4-cis polybutadiene prepared in the examples and the comparative examples was each dissolved for 30 minutes in tetrahydrofuran (THF) under a condition of 40°C, and was loaded and passed through gel permeation chromatography (GPC). Herein, two PLgel Olexis (trade name) columns and a PLgel mixed-C column manufactured by Polymer Laboratories were combined and used as the column. In addition, mixed bed-type columns were all used as the newly replaced column, and polystyrene was used as a gel permeation chromatography (GPC) standard material.

2) Mooney Viscosity and -S/R Value

For the 1,4-cis polybutadiene prepared in the examples and the comparative examples, Mooney viscosity (MV) was measured using a Large Rotor of MV2000E manufactured by Monsanto under a condition of Rotor Speed 2$\pm$0.02 rpm at 100°C. Herein, the used sample was left unattended for 30 minutes or longer at room temperature (23$\pm$5°C), 27$\pm$3 g thereof was collected, and inside a die cavity is filled with the sample, and Mooney viscosity was measured while operating a Platen and applying Torque.

In addition, changes in the Mooney viscosity appearing while releasing Torque were observed when measuring the Mooney viscosity, and the -S/R value was determined from the slope.

3) Cis-1,4 Bond Content

For the 1,4-cis polybutadiene prepared in the examples and the comparative examples, Fourier Transform Infrared Spectroscopy analyses were carried out, and cis-1,4 bond content in the 1,4-cis polybutadiene was obtained from the results.

[Table 1]

| | Chain transfer agent-Containing Mixture Preparation[1)] | Catalyst Composition Preparation | | | | | Polymerization Reaction | | Conversion Rate (%) | Catalytic Activity (kg [Polymer]/mol [Nd] - h) | Physical Property Evaluation | | | | | |
|---|---|---|---|---|---|---|---|---|---|---|---|---|---|---|---|---|
| | DIBAH (mmol) | Nd-Based Compound (mmol) | MMAO (mmol) | DEAC (mmol) | Hexane (mmol) | DIBAH (mmol) | Temp. (°C) | Time (min) | | | Mn ($x10^5$ g/mol) | Mw ($x10^5$ g/mol) | PDI | MV | -S/R | Cis-1,4 Bond Content (%) |
| Ex.1 | 0.25 | Prep. 1 0.08 | 8.0 | 0.18 | 80 | - | 70 | 10 | 100 | 13,194 | 8.9 | 22.2 | 2.5 | 88.6 | 1.0521 | 96.8 |
| Ex.2 | 0.66 | Prep. 1 0.08 | 4.0 | 0.18 | 80 | - | 70 | 10 | 100 | 13,194 | 6.7 | 15.9 | 2.38 | 75.7 | 1.0456 | 96.2 |
| Ex.3 | 1.0 | Prep.2 0.04 | 1.2 | 0.09 | 40 | - | 70 | 10 | 100 | 26,338 | 4.3 | 9.9 | 2.31 | 43.5 | 1.0423 | 98.4 |
| Ex.4 | 1.0 | Prep.2 0.04 | 0.8 | 0.09 | 40 | - | 70 | 10 | 100 | 26,338 | 4.2 | 10.2 | 2.41 | 59.8 | 1.0786 | 98.5 |
| Comp. 1 | - | Prep. 1 0.24 | - | 0.55 | 120 | 3.0 | 70 | 30 | 88 | 733 | 1.9 | 6.2 | 3.24 | 46.0 | 0.6529 | 96.4 |
| Comp. 2 | - | Prep. 2 0.24 | - | 0.55 | 120 | 3.0 | 70 | 30 | 86 | 717 | 2.1 | 9.0 | 4.34 | 45.5 | 0.6556 | 97.8 |

*In Table 1, 'Ex.' means Example, 'Comp.' means Comparative example, 'Prep.' means Preparation example, and 'Temp.' means Temperature.

[Table 2]

| | Chain transfer agent-Containing Mixture Preparation[1)] | Catalyst Composition Preparation | | | | | Polymerization Reaction | | Conversion Rate (%) | Cataly tic Activi ty (kg[Polymer]/mol [Nd] -h) | Physical Property Evaluation | | | | | |
|---|---|---|---|---|---|---|---|---|---|---|---|---|---|---|---|---|
| | DIBAH (mmol) | Nd-Based Compound (mmol) | MMAO (mmol) | DEAC (mmol) | Hexane (mmol) | DIBAH (mmol) | Temp. (°C) | Time (min) | | | Mn ($x10^5$ g/ mol ) | Mw ($x10^5$ g/ mol ) | PDI | MV | -S/R | Cis-1,4 Bond Content (%) |
| Ex.5 | 0.92 | Prep. 2 0.08 | 8.0 | 0.18 | 80 | - | 30 | 40 | 100 | 4,688 | 3.1 | 7.4 | 2.35 | 39.5 | 1.0306 | 96.0 |
| Ex.6 | 0.87 | Prep. 2 0.08 | 8.0 | 0.18 | 80 | - | 30 | 40 | 100 | 4,688 | 3.2 | 8.0 | 2.46 | 46.1 | 1.0122 | 95.6 |
| Ex.7 | 0.83 | Prep. 2 0.08 | 8.0 | 0.18 | 80 | - | 30 | 40 | 100 | 4,688 | 3.4 | 8.3 | 2.41 | 50.5 | 1.0446 | 96.1 |
| Comp. 3 | - | Prep. 2 0.20 | - | 0.46 | 100 | 2.04 | 70 | 60 | 96 | 1,200 | 2.0 | 6.3 | 3.06 | 33.5 | 0.8563 | 96.5 |
| Comp. 4 | - | Prep. 2 0.20) | - | 0.46 | 100 | 1.82 | 70 | 60 | 91 | 1,318 | 2.4 | 7.7 | 3.28 | 43.8 | 0.8548 | 97.4 |

*In Table 2, 'Ex.' means Example, 'Comp.' means Comparative example, 'Prep.' means Preparation example, and 'Tenp.' means Temperature.

In Tables 1 and 2, preparation of a mixture containing a chain transfer agent of 1) means preparation of a mixture by mixing a chain transfer agent and a diene-based monomer.

Table 1 compares polymer conversion rates, catalytic activity, and cis-1,4 bond content in the prepared polymers, molecular weight distribution and linearity depending on the content of the MMAO and the DIBAH, and the order of the DIBAH introduction.

As can be seen from Table 1, the polymers of Examples 1 to 4 exhibited significantly enhanced polymer conversion rates and catalytic activity compared to Comparative Examples 1 and 2.

When specifically examined, in Examples 1 to 4, the polymerization time was reduced to 1/3 at the same polymerization temperature even when using the Nd-based main catalyst compound in a small amount of approximately 1/6 to 1/3 compared to Comparative Examples 1 and 2. In addition, in Examples 1 to 4, a 100% polymer conversion rate was obtained even when reducing the amount of the main catalyst and the polymerization time. Meanwhile, in Comparative Examples 1 and 2, low polymer conversion rates of approximately 86% to 88% were obtained despite that the amount of the main catalyst increased by 3 times to 6 times, and the polymerization time increased by 3 times compared to Examples 1 to 4.

In addition, in Examples 1 to 4, catalytic activity was enhanced up to 15 times to 35 times when compared to Comparative Examples 1 and 2.

Furthermore, the 1,4-cis polybutadiene prepared in Examples 1 to 4 exhibited narrower molecular weight distribution compared to Comparative Examples 1 and 2. Specifically, whereas the 1,4-cis polybutadiene of Examples 1 to 4 had PDI in a range of 2.3 to 2.5 with a molecular weight distribution range of 2.5 or less, the polymers of Comparative Examples 1 and 2 had PDI of 3.24 and 4.34, respectively, and exhibited significantly increased molecular weight distribution compared to Examples 1 to 4.

In addition, Table 2 compares polymer conversion rates, catalytic activity, and cis-1,4 bond content in the prepared 1,4-cis polybutadiene, molecular weight distribution and linearity depending on the order of the DIBAH introduction while varying the DIBAH content and the polymerization temperature.

Specifically, as can be seen from Table 2, Examples 5 to 7 had very high catalytic activity, and polymerization was readily carried out in a short period of time even at a low temperature (30°C). Meanwhile, in Comparative Examples 3 and 4, the polymerization conversion rate did not reach 100% even when polymerization was carried out for 60 minutes at 70°C.

In addition, the 1,4-cis polybutadiene prepared in Examples 5 to 7 had -S/R of 1 or greater, a value increased by 20% or greater compared to Comparative Examples 3 and 4. From this result, it may be predicted that the 1,4-cis polybutadiene of Example 5 to 7 had very high linearity, and as a result, when used in tires, rolling resistance declines and fuel efficiency properties are capable of being enhanced.

## Claims

**1.** A catalyst composition comprising:

a lanthanide rare earth element-containing compound;
modified methylaluminoxane;
a halogen compound; and
an aliphatic hydrocarbon-based solvent, and
wherein the lanthanide rare earth element-containing compound comprises a neodymium compound of the following Chemical Formula 1:

[Chemical Formula 1]

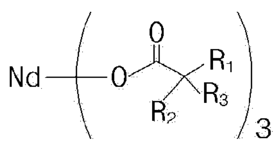

in Chemical Formula 1, $R_1$ to $R_3$ are each independently a hydrogen atom, or a linear or branched alkyl group having 1 to 12 carbon atoms,
wherein the modified methylaluminoxane is a compound of the following Chemical Formula 2:

[Chemical Formula 2]

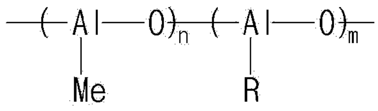

in Chemical Formula 2, R is a hydrocarbon group having 2 to 20 carbon atoms, m and n are each an integer of 2 or greater, and Me means a methyl group, and wherein the catalyst composition does not comprise diisobutylaluminum hydride.

**2.** The catalyst composition of Claim 1, wherein, in the modified methylaluminoxane, 50 mol% to 90 mol% of a methyl group of the methylaluminoxane is substituted with a hydrocarbon group having 2 to 20 carbon atoms.

**3.** The catalyst composition of Claim 2, wherein the hydrocarbon group is a linear or branched alkyl group having 2 to 10 carbon atoms.

**4.** The catalyst composition of Claim 1, wherein the modified methylaluminoxane comprises trimethylaluminum; and a mixed alkyl group derived from a trialkylaluminum other than trimethylaluminum, and the trialkylaluminum comprises any one or a mixture of two or more selected from the group consisting of triisobutylaluminum, triethylaluminum, trihexylaluminum and trioctylaluminum.

**5.** The catalyst composition of Claim 1, wherein the lanthanide rare earth element-containing compound comprises a neodymium compound in which, in Chemical Formula 1, $R_1$ is a linear or branched alkyl group having 6 to 12 carbon atoms, and $R_2$ and $R_3$ are each independently a hydrogen atom or a linear or branched alkyl group having 2 to 8 carbon atoms, but $R_2$ and $R_3$ are not both hydrogen atoms at the same time.

**6.** The catalyst composition of Claim 1, wherein the lanthanide rare earth element-containing compound comprises any one or a mixture of two or more selected from the group consisting of $Nd(neodecanoate)_3$, Nd(2,2-diethyl decanoate)$_3$, Nd(2,2-dipropyl decanoate)$_3$, Nd(2,2-dibutyl decanoate)$_3$, Nd(2,2-dihexyl decanoate)$_3$, Nd(2,2-dioctyl decanoate)$_3$, Nd(2-ethyl-2-propyl decanoate)$_3$, Nd(2-ethyl-2-butyl decanoate)$_3$, Nd(2-ethyl-2-hexyl decanoate)$_3$, Nd(2-propyl-2-butyl decanoate)$_3$, Nd(2-propyl-2-hexyl decanoate)$_3$, Nd(2-propyl-2-isopropyl decanoate)$_3$, Nd(2-butyl-2-hexyl decanoate)$_3$, Nd(2-hexyl-2-octyl decanoate)$_3$, Nd(2-t-butyl decanoate)$_3$, Nd(2,2-diethyl octanoate)$_3$, Nd(2,2-dipropyl octanoate)$_3$, Nd(2,2-dibutyl octanoate)$_3$, Nd(2,2-dihexyl octanoate)$_3$, Nd(2-ethyl-2-propyl octanoate)$_3$, Nd(2-ethyl-2-hexyl octanoate)$_3$, Nd(2,2-diethyl nonanoate)$_3$, Nd(2,2-dipropyl nonanoate)$_3$, Nd(2,2-dibutyl nonanoate)$_3$, Nd(2,2-dihexyl nonanoate)$_3$, Nd(2-ethyl-2-propyl nonanoate)$_3$ and Nd(2-ethyl-2-hexyl nonanoate)$_3$.

**7.** The catalyst composition of Claim 1, wherein the aliphatic hydrocarbon-based solvent comprises any one or a mixture of two or more selected from the group consisting of linear, branched or cyclic aliphatic hydrocarbon having 5 to 20 carbon atoms.

**8.** The catalyst composition of Claim 1, wherein the aliphatic hydrocarbon-based solvent comprises any one selected from the group consisting of hexane, cyclohexane and a mixture thereof.

**9.** The catalyst composition of Claim 1, wherein the halogen compound comprises any one or a mixture of two or more selected from the group consisting of fluorine ($F_2$), chlorine ($Cl_2$), bromine ($Br_2$, iodine ($I_2$), iodine monochloride, iodine monobromide, iodine trichloride, iodine pentafluoride, iodine monofluoride, iodine trifluoride, hydrogen fluoride, hydrogen chloride, hydrogen bromide, hydrogen iodide, organic halides, non-metal halides, metal halides and organic metal halides.

**10.** The catalyst composition of Claim 1, which comprises the modified methylaluminoxane in a molar ratio of 5 to 200 with respect to 1 mol of the lanthanide rare earth element-containing compound.

**11.** The catalyst composition of Claim 1, which comprises the modified methylaluminoxane in 5 mol to 200 mol, the halogen compound in 1 mol to 10 mol and the aliphatic hydrocarbon-based solvent in 20 mol to 20,000 mol with respect to 1 mol of the lanthanide rare earth element-containing compound.

**12.** The catalyst composition of Claim 1, which is a pre-mixture of the lanthanide rare earth element-containing compound, the modified methylaluminoxane, the halogen compound and the aliphatic hydrocarbon-based solvent.

**13.** A method for preparing the catalyst composition of any one of Claims 1 to 12, the method comprising mixing a lanthanide rare earth element-containing compound, modified methylaluminoxane, a halogen compound and an aliphatic hydrocarbon-based solvent, and then heat treating the resultant at a temperature of 0°C to 60°C.

**14.** A method for preparing the catalyst composition of any one of Claims 1 to 12, the method comprising mixing a lanthanide rare earth element-containing compound, modified methylaluminoxane, and an aliphatic hydrocarbon-based solvent, first heat treating the result at a temperature of 10°C to 60°C, introducing a halogen compound to the resultantly obtained mixture, and second heat treating the result in a temperature range of 0°C to 60°C.

**15.** A method for preparing a conjugated-diene based polymer using the catalyst composition of any one of claims 1 to 12.

**Patentansprüche**

**1.** Katalysatorzusammensetzung umfassend:

eine Lanthanoid-Seltenerdelement enthaltende Verbindung;
modifiziertes Methylaluminoxan;
eine Halogenverbindung und
ein Lösungsmittel auf Basis eines aliphatischen Kohlenwasserstoffs und
wobei die Lanthanoid-Seltenerdelement enthaltende Verbindung eine Neodymverbindung der nachstehenden Chemischen Formel 1 umfasst:

[Chemische Formel 1]

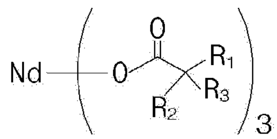

in der Chemischen Formel 1 sind $R_1$ bis $R_3$ jeweils unabhängig ein Wasserstoffatom oder eine geradkettige oder verzweigte Alkylgruppe mit 1 bis 12 Kohlenstoffatomen,
wobei das modifizierte Methylaluminoxan eine Verbindung der nachstehenden Chemischen Formel 2 ist:

[Chemische Formel 2]

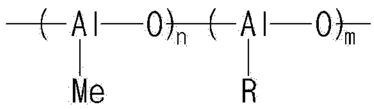

in der Chemischen Formel 2 ist R eine Kohlenwasserstoffgruppe mit 2 bis 20 Kohlenstoffatomen, m und n sind jeweils eine ganze Zahl von 2 oder grösser, und Me bedeutet eine Methylgruppe, und
wobei die Katalysatorzusammensetzung kein Diisobutylaluminumhydrid umfasst.

**2.** Katalysatorzusammensetzung gemäss Anspruch 1, wobei, in dem modifizierten Methylaluminoxan 50 bis 90 mol-% einer Methylgruppe des Methylaluminoxans mit einer Kohlenwasserstoffgruppe mit 2 bis 20 Kohlenstoffatomen substituiert sind.

**3.** Katalysatorzusammensetzung gemäss Anspruch 2, wobei die Kohlenwasserstoffgruppe eine geradkettige oder verzweigte Alkylgruppe mit 2 bis 10 Kohlenstoffatomen ist.

**4.** Katalysatorzusammensetzung gemäss Anspruch 1, wobei das modifizierte Methylaluminoxan Trimethylaluminum und eine gemischte Alkylgruppe, die von einem anderen Trialkylaluminum als Trimethylaluminum abgeleitet ist, umfasst und das Trialkylaluminum irgendeines oder eine Mischung von zwei oder mehreren, ausgewählt aus der Gruppe bestehend aus Triisobutylaluminum, Triethylaluminum, Trihexylaluminum und Trioctylaluminum, umfasst.

**5.** Katalysatorzusammensetzung gemäss Anspruch 1, wobei die Lanthanoid-Seltenerdelement enthaltende Verbindung eine Neodymverbindung umfasst, in welcher in der Chemischen Formel 1 $R_1$ eine geradkettige oder verzweigte Alkylgruppe mit 6 bis 12 Kohlenstoffatomen ist und $R_2$ und $R_3$ jeweils unabhängig ein Wasserstoffatom oder eine geradkettige oder verzweigte Alkylgruppe mit 2 bis 8 Kohlenstoffatomen sind, aber $R_2$ und $R_3$ nicht beide gleichzeitig Wasserstoffatome sind.

**6.** Katalysatorzusammensetzung gemäss Anspruch 1, wobei die Lanthanoid-Seltenerdelement enthaltende Verbindung irgendeines oder eine Mischung von zwei oder mehreren, ausgewählt aus der Gruppe bestehend aus Nd(Neodecanoat)$_3$, Nd(2,2-Diethyldecanoat)$_3$, Nd(2,2-Dipropyldecanoat)$_3$, Nd(2,2-Dibutyldecanoat)$_3$, Nd(2,2-Dihexyldecanoat)$_3$, Nd(2,2-Dioctyldecanoat)$_3$, Nd(2-Ethyl-2-propyldecanoat)$_3$, Nd(2-Ethyl-2-butyldecanoat)$_3$, Nd(2-Ethyl-2-hexyldecanoat)$_3$, Nd(2-Propyl-2-butyldecanoat)$_3$, Nd(2-Propyl-2-hexyldecanoat)$_3$, Nd(2-Propyl-2-isopropyldecanoat)$_3$, Nd(2-Butyl-2-hexyldecanoat)$_3$, Nd(2-Hexyl-2-octyldecanoat)$_3$, Nd(2-t-Butyldecanoat)$_3$, Nd(2,2-Diethyloctanoat)$_3$, Nd(2,2-Dipropyloctanoat)$_3$, Nd(2,2-Dibutyloctanoat)$_3$, Nd(2,2-Dihexyloctanoat)$_3$, Nd(2-Ethyl-2-propyloctanoat)$_3$, Nd(2-Ethyl-2-hexyloctanoat)$_3$, Nd(2,2-Diethylnonanoat)$_3$, Nd(2,2-Dipropylnonanoat)$_3$, Nd(2,2-Dibutylnonanoat)$_3$, Nd(2,2-Dihexylnonanoat)$_3$, Nd(2-Ethyl-2-propylnonanoat)$_3$ und Nd(2-Ethyl-2-hexylnonanoat)$_3$ umfasst.

**7.** Katalysatorzusammensetzung gemäss Anspruch 1, wobei das Lösungsmittel auf Basis eines aliphatischen Kohlenwasserstoffs irgendeines oder eine Mischung von zwei oder mehreren, ausgewählt aus der Gruppe bestehend aus geradkettigem, verzweigtem oder cyclischem aliphatischen Kohlenwasserstoff mit 5 bis 20 Kohlenstoffatomen umfasst.

**8.** Katalysatorzusammensetzung gemäss Anspruch 1, wobei das Lösungsmittel auf Basis eines aliphatischen Kohlenwasserstoffs irgendeines, ausgewählt aus der Gruppe bestehend aus Hexan, Cyclohexan und einer Mischung davon, umfasst.

**9.** Katalysatorzusammensetzung gemäss Anspruch 1, wobei die Halogenverbindung irgendeines oder eine Mischung von zwei oder mehreren, ausgewählt aus der Gruppe bestehend aus Fluor ($F_2$), Chlor ($Cl_2$), Brom ($Br_2$), Iod ($I_2$), Iodmonochlorid, Iodmonobromid, Iodtrichlorid, Iodpentafluorid, Iodmonofluorid, Iodtrifluorid, Fluorwasserstoff, Chlorwasserstoff, Bromwasserstoff, Iodwasserstoff, organischen Halogeniden, Nichtmetallhalogeniden, Metallhalogeniden und organischen Metallhalogeniden, umfasst.

**10.** Katalysatorzusammensetzung gemäss Anspruch 1, die das modifizierte Methylaluminoxan in einem Molverhältnis von 5 bis 200, bezogen auf 1 mol der Lanthanoid-Seltenerdelement enthaltenden Verbindung, umfasst.

**11.** Katalysatorzusammensetzung gemäss Anspruch 1, die 5 bis 200 mol des modifizierten Methylaluminoxans, die 1 bis 10 mol der Halogenverbindung und 20 bis 20.000 mol des Lösungsmittels auf Basis eines aliphatischen Kohlenwasserstoffs, bezogen auf 1 mol der Lanthanoid-Seltenerdelement enthaltenden Verbindung, umfasst.

**12.** Katalysatorzusammensetzung gemäss Anspruch 1, die ein Vorgemisch der Lanthanoid-Seltenerdelement enthaltenden Verbindung, des modifizierten Methylaluminoxans, der Halogenverbindung und des Lösungsmittels auf Basis eines aliphatischen Kohlenwasserstoffs ist.

**13.** Verfahren zur Herstellung der Katalysatorzusammensetzung gemäss irgendeinem der Ansprüche 1 bis 12, wobei das Verfahren das Mischen einer Lanthanoid-Seltenerdelement enthaltenden Verbindung, eines modifizierten Methylaluminoxans, einer Halogenverbindung und eines Lösungsmittels auf Basis eines aliphatischen Kohlenwasserstoffs und dann Wärmebehandeln der resultierenden Mischung bei einer Temperatur von 0 bis 60°C umfasst.

**14.** Verfahren zur Herstellung der Katalysatorzusammensetzung gemäss irgendeinem der Ansprüche 1 bis 12, wobei das Verfahren das Mischen einer Lanthanoid-Seltenerdelement enthaltenden Verbindung, eines modifizierten Methylaluminoxans und eines Lösungsmittels auf Basis eines aliphatischen Kohlenwasserstoffs, eine erste Wärmebehandlung der resultierenden Mischung bei einer Temperatur von 10 bis 60°C, Einführen einer Halogenverbindung in die resultierende erhaltene Mischung und eine zweite Wärmebehandlung des Resultats in einem Temperaturbereich von 0 bis 60°C umfasst.

**15.** Verfahren zur Herstellung eines Polymers auf Basis eines konjugierten Diens unter Verwendung der Katalysatorzusammensetzung gemäss irgendeinem der Ansprüche 1 bis 12.

## Revendications

**1.** Composition de catalyseur comprenant :

un composé contenant un élément des terres rares lanthanides ;
un méthylaluminoxane modifié ;
un composé halogéné ; et
un solvant à base d'hydrocarbure aliphatique, et
dans laquelle le composé contenant un élément des terres rares lanthanides comprend un composé de néodyme de formule chimique 1 suivante :

[Formule chimique 1]

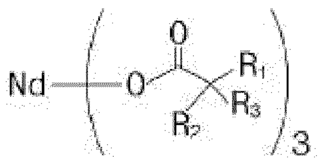

dans la formule chimique 1, $R_1$ à $R_3$ sont chacun indépendamment un atome d'hydrogène, ou un groupe alkyle linéaire ou ramifié ayant 1 à 12 atomes de carbone,
dans laquelle le méthylaluminoxane modifié est un composé de formule chimique 2 suivante :

[Formule chimique 2]

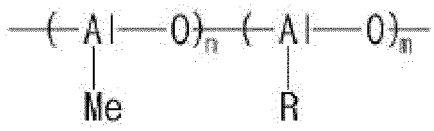

dans la formule chimique 2, R est un groupe hydrocarboné ayant 2 à 20 atomes de carbone, m et n sont chacun un nombre entier égal à 2 ou plus, et Me indique un groupe méthyle, et
dans laquelle la composition de catalyseur ne comprend pas d'hydrure de diisobutylaluminium.

**2.** Composition de catalyseur selon la revendication 1, dans laquelle, dans le méthylaluminoxane modifié, 50 % en mole à 90 % en mole d'un groupe méthyle du méthylaluminoxane sont remplacés par un groupe hydrocarboné ayant 2 à 20 atomes de carbone.

**3.** Composition de catalyseur selon la revendication 2, dans laquelle le groupe hydrocarboné est un groupe alkyle linéaire ou ramifié ayant 2 à 10 atomes de carbone.

**4.** Composition de catalyseur selon la revendication 1, dans laquelle le méthylaluminoxane modifié comprend du triméthylaluminium ; et un groupe alkyle mixte dérivé d'un trialkylaluminium autre que le triméthylaluminium, et le trialkylaluminium comprend l'un quelconque ou un mélange de deux ou plus de deux sélectionnés dans le groupe constitué du triisobutylaluminium, du triéthylaluminium, du trihexylaluminium et du trioctylaluminium.

**5.** Composition de catalyseur selon la revendication 1, dans laquelle le composé contenant un élément des terres rares lanthanides comprend un composé de néodyme dans lequel, dans la formule chimique 1, $R_1$ est un groupe alkyle linéaire ou ramifié ayant 6 à 12 atomes de carbone, et $R_2$ et $R_3$ sont chacun indépendamment un atome d'hydrogène ou un groupe alkyle linéaire ou ramifié ayant 2 à 8 atomes de carbone, mais $R_2$ et $R_3$ ne sont pas tous les deux des atomes d'hydrogène en même temps.

**6.** Composition de catalyseur selon la revendication 1, dans laquelle le composé contenant un élément des terres rares lanthanides comprend l'un quelconque ou un mélange de deux ou plus de deux sélectionnés dans le groupe

constitué du Nd(néodécanoate)$_3$, du Nd(2,2-diéthyl décanoate)$_3$, du Nd(2,2-dipropyl décanoate)$_3$, du Nd(2,2-dibutyl décanoate)$_3$, du Nd(2,2-dihexyl décanoate)$_3$, du Nd(2,2-dioctyl décanoate)$_3$, du Nd(2-éthyl-2-propyl décanoate)$_3$, du Nd(2-éthyl-2-butyl décanoate)$_3$, du Nd(2-éthyl-2-hexyl décanoate)$_3$, du Nd(2-propyl-2-butyl décanoate)$_3$, du Nd(2-propyl-2-hexyl décanoate)$_3$, du Nd(2-propyl-2-isopropyl décanoate)$_3$, du Nd(2-butyl-2-hexyl décanoate)$_3$, du Nd(2-hexyl-2-octyl décanoate)$_3$, du Nd(2-t-butyl décanoate)$_3$, du Nd(2,2-diéthyl octanoate)$_3$, du Nd(2,2-dipropyl octanoate)$_3$, du Nd(2,2-dibutyl octanoate)$_3$, du Nd(2,2-dihexyl octanoate)$_3$, du Nd(2-éthyl-2-propyl octanoate)$_3$, du Nd(2-éthyl-2-hexyl octanoate)$_3$, du Nd(2,2-diéthyl nonanoate)$_3$, du Nd(2,2-dipropyl nonanoate)$_3$, du Nd(2,2-dibutyl nonanoate)$_3$, du Nd(2,2-dihexyl nonanoate)$_3$, du Nd(2-éthyl-2-propyl nonanoate)$_3$ et du Nd(2-éthyl-2-hexyl nonanoate)$_3$.

**7.** Composition de catalyseur selon la revendication 1, dans laquelle le solvant à base d'hydrocarbure aliphatique comprend l'un quelconque ou un mélange de deux ou plus de deux sélectionnés dans le groupe constitué d'un hydrocarbure aliphatique linéaire, ramifié ou cyclique ayant 5 à 20 atomes de carbone.

**8.** Composition de catalyseur selon la revendication 1, dans laquelle le solvant à base d'hydrocarbure aliphatique comprend l'un quelconque sélectionné dans le groupe constitué de l'hexane, du cyclohexane et d'un mélange de ceux-ci.

**9.** Composition de catalyseur selon la revendication 1, dans laquelle le composé halogéné comprend l'un quelconque ou un mélange de deux ou plus de deux sélectionnés dans le groupe constitué du fluor ($F_2$), du chlore ($Cl_2$), du brome ($Br_2$), de l'iode ($I_2$), du monochlorure d'iode, du monobromure d'iode, du trichlorure d'iode, du pentafluorure d'iode, du monofluorure d'iode, du trifluorure d'iode, du fluorure d'hydrogène, du chlorure d'hydrogène, du bromure d'hydrogène, de l'iodure d'hydrogène, des halogénures organiques, des halogénures non métalliques, des halogénures métalliques et des halogénures métalliques organiques.

**10.** Composition de catalyseur selon la revendication 1, qui comprend le méthylaluminoxane modifié à un rapport molaire de 5 à 200 par rapport à 1 mole du composé contenant un élément des terres rares lanthanides.

**11.** Composition de catalyseur selon la revendication 1, qui comprend le méthylaluminoxane modifié de 5 moles à 200 moles, le composé halogéné de 1 mole à 10 moles et le solvant à base d'hydrocarbure aliphatique de 20 moles à 20 000 moles par rapport à 1 mole du composé contenant un élément des terres rares lanthanides.

**12.** Composition de catalyseur selon la revendication 1, qui est un prémélange du composé contenant un élément des terres rares lanthanides, du méthylaluminoxane modifié, du composé halogéné et du solvant à base d'hydrocarbure aliphatique.

**13.** Procédé pour préparer la composition de catalyseur selon l'une quelconque des revendications 1 à 12, le procédé comprenant le mélange d'un composé contenant un élément des terres rares lanthanides, d'un méthylaluminoxane modifié, d'un composé halogéné et d'un solvant à base d'hydrocarbure aliphatique, et ensuite le traitement thermique de ce qui est obtenu à une température de 0 °C à 60 °C.

**14.** Procédé pour préparer la composition de catalyseur selon l'une quelconque des revendications 1 à 12, le procédé comprenant le mélange d'un composé contenant un élément des terres rares lanthanides, d'un méthylaluminoxane modifié, et d'un solvant à base d'hydrocarbure aliphatique, un premier traitement thermique de ce qui est obtenu à une température de 10 °C à 60 °C, l'introduction d'un composé halogéné dans le mélange ainsi obtenu, et un second traitement thermique de ce qui est obtenu dans une plage de températures allant de 0 °C à 60 °C.

**15.** Procédé pour préparer un polymère à base de diènes conjugués en utilisant la composition de catalyseur selon l'une quelconque des revendications 1 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102014162933
- KR 102014162934
- KR 102015161323